# EUROPEAN PATENT APPLICATION

(11) **EP 3 299 037 A1**
(43) Date of publication of application: **28.03.2018**
(21) Application number: 16190715.9
(22) Date of filing: 27.09.2016
(51) Int. Cl.: A61L 27/04, A61L 27/24, A61L 27/34, A61L 27/50, A61L 27/58

(54) **BARRIER SYSTEM AND METHOD OF FORMING A BARRIER SYSTEM, A METHOD OF REGENERATING A BONE AND A REINFORCEMENT MEMBER**

(71) Applicant: Regedent AG, 8008 Zürich (CH)
(72) Inventor: Lussi, Jost, 6370 Oberdorf (CH); Früh, Herbert, 8906 Bonstetten (CH); De Gruttola, Sandro, 5000 Aarau (CH); Löffler, Jörg F, 8606 Greifensee (CH); Uggowitzer, Peter J., 8913 Ottenbach (CH)
(74) Representative: Hepp Wenger Ryffel AG

(57) **Abstract**

A barrier system (1) for guided bone and tissue regeneration. The barrier system (1) comprises a membrane, preferably a bioresorbable membrane (2) and at least one bioresorbable reinforcement member (3). The reinforcement member (3) comprises or is made of a bioresorbable polymer, preferably a synthetic polymer and/or a bioresorbable metal, preferably a magnesium alloy.

## Description

The present application is directed to a barrier system for guided bone and tissue regeneration, to a method of forming such a barrier system, to a method of regenerating a bone and tissue as well as to a reinforcement member for barrier systems for guided bone and tissue regeneration, according to the independent claims.

After surgery regeneration of bone defects often has to take place. For example in oral constructive surgery, bone defects may arise due to tooth extraction. It is desired that only bone cells regenerate the bone defect and not surrounding tissue cells. The rapidly growing tissue cells should therefore be prevented from invading the bone defect. Barrier systems are used which are preventing the tissue cells from migrating into the bone defect to avoid having an inferior bone regeneration.

Different membranes have been suggested to be used in barrier systems for guided bone regeneration, for example PTFE or resorbable collagen membranes. Resorbable membranes have the advantage that the membrane does not have to be removed (e.g. by the dentist) after bone regeneration. Therewith, irritation of the tissue is prevented which might occur if a membrane needs to be removed which has somehow connected to the surrounding tissue during the healing phase.

Barrier membranes are often used in combination with bone graft materials in particulate form. Particulate graft material is not capable of maintaining the augmented space during healing due to its mobility upon application of pressure by soft tissue contraction and / or tongue movement. The placement of a membrane on top of such graft material prevents undesired dislocation of graft particulates and therefore loss of augmented bone volume. The membrane is usually held in place either by pinning/screwing it to the bone or suturing to the soft tissue.

In case a larger bone volume is to be augmented and protected with a membrane it is desirable to use a barrier membrane with a deformable reinforcement element. The reinforcement element can be pre-shaped to the anatomically condition of the surgical site. Adequate mechanical properties of the reinforcement element prevent the membrane from being deformed during healing and therefore reduced bone augmentation volume.

EP 2 959 862 suggests providing a membrane with two layers and a flexible titanium reinforcement.

The titanium reinforcement has to be removed from the treatment site after bone regeneration. A removal of a rigid element requires an additional surgical intervention, is cumbersome and also bears the risk of tissue irritation and therefore of inferior bone regeneration. Hence, there is a need to provide a barrier system for bone regeneration which avoids at least some of the disadvantages of the prior art. In particular it is desirable to have a barrier system with a reduced risk of tissue irritation and site morbidity.

According to the present invention, this problem is solved with a barrier system according to the independent claim. The barrier system for guided bone and tissue regeneration comprises a membrane and at least one bioresorbable reinforcement member. The membrane is preferably also bioresorbable. The reinforcement member comprises or is made of a bioresorbable polymer, preferably a synthetic polymer and/or a bioresorbable metal, preferably a magnesium alloy.

As at least the reinforcement member is bioresorbable there is no need of a removal of rigid elements after tissue bone regeneration. Therefore, tissue irritation and an additional surgery can be avoided. In case both parts are bioresorbable, no part has to be removed from the surgical site further decreasing the risk of tissue irritation and site morbidity.

Polycaprolactone might be used as a resorbable polymer for the reinforcement member(s). Polycaprolactone is a biocompatible synthetic polymer which can be deformed by gentle heating in order to pre-shape the reinforcement member(s). In case the membrane is attached to the reinforcement member, the membrane can be pre-shaped before implantation. Alternatively, a bioresorbable metal alloy with adequate mechanical properties, such as a magnesium alloy or an iron-based alloy, might be used as a material for the reinforcement member(s).

The membrane is preferably a bioresorbable collagen membrane.

Collagen membranes have been shown to provide a reliable barrier function and a good biocompatibility as well as bioresorbability. Further, such collagen membranes can be formed such as to be bioresorbed not until after a desired time, i.e. not until after bone regeneration has been sufficiently progressed. Such a collagen membrane might be formed by adequate processing of native collagen tissues from animal species, such as porcine pericardium.

Another preferred example of a bioresorbable membrane is a reconstituted collagen membrane. To form such a reconstituted membrane, collagen is first separated into individual fibers. The fibers are then crosslinked again to form the reconstituted membrane. Such reconstituted membranes allow the formation of membranes with the desired dimensions. They might be thicker or thinner as native tissue membranes. Further, the bioresorbability can be well defined. Formation of reconstituted collagen membranes is described in EP 0 693 523, whose respective disclosure is incorporated herein by reference.

Preferably, the collagen membrane is a collagenase resistant membrane. Collagenases are enzymes which break peptide bonds in collagen and results in faster collagen membrane degradation. By providing a collagenase resistant collagen membrane, break of bonds and therewith destabilization and/or premature resorption of the membrane is avoided.
Alternatively, the membrane might be formed of other materials such as a resorbable metal alloy (e.g. a magnesium alloy) or a resorbable polymer such a Polylactid-co-Glycolid (PLGA).

The membrane might also be stimulating such as specifically enhancing bone regeneration or enhancing soft tissue healing. Such membranes might be synthetic or from a biological source. It is also possible to combine membrane materials. It is e.g. possible that the side of the membrane being in contact with the bone enhances bone regeneration and the side of the membrane in contact with the soft tissue enhances wound healing.

It is also possible to use non-bioresorbable membranes such as PTFE membranes with bioresorbable reinforcement members in a barrier system according to the invention.

The reinforcement member is preferably embedded, i.e. covered on all sides, in the membrane. Embedding of the reinforcement member is preferably performed with and during formation of a reconstituted collagen membrane. The reinforcement member can be embedded between layers of collagen fibers which are constituted to form the reinforced membrane.

For example, a layer of collagen fibers is first deposited in a mold, optionally providing the desired membrane dimensions (length and width), then a reinforcement member is put on top of this not yet crosslinked collagen layer. Another layer of collagen is deposited on top and the system is compressed to its desired density/thickness and exposed to adequate conditions to achieve the desired crosslinking between the collagen fibers.

The thickness of the reinforcement member is preferably kept low such that the barrier system preferably has a total thickness of less than 1 mm, more preferably less than 0.5 mm.

The ratio between the thicknesses of the reinforcement member(s) and the membrane is preferably around 1:2. The reinforcement member(s) might have a thickness of about 0.1 - 0.3 mm and the membrane of about 0.2 - 0.8 mm. However, the reinforcement member(s) and/or the membrane might be thicker. The reinforcement member(s) might be up to 0.5 mm, the membrane up to 2 mm depending on the specific treatment situation.

Alternatively, the reinforcement member(s) might be mechanically connected to the membrane. The reinforcement member(s) is preferably mechanically connected to a surface of the membrane. The reinforcement member(s) might for example be glued, pinned, soldered or sutured to the bioresorbable membrane.

By providing a membrane attached to the reinforcement member(s) (either through embedding or mechanical connection), the reinforced barrier membrane can be stored as a single product. Further, the time used for the implantation can be reduced as only one device has to be implanted. It is also possible to pre-shape the membrane into the desired form before implantation.

It is also possible, that that reinforcement member(s) and the bioresorbable membrane are not connected to each other but rather supplied as separate parts of the barrier system. In such a case, the user might first place a desirably shaped reinforcement member at the defect site, which is then covered by the membrane. The membrane is kept in place either by fixing it (e.g. by pins or sutures) to a suitably designed reinforcement member or to the surrounding tissue or alternatively by the pressure of the soft tissue after it is closed over the membrane. The different parts of the barrier system are therefore brought together by the user in situ.

In case a bioresorbable membrane is used, the membrane is preferably adapted to not be resorbed before 8-12 weeks in a body of a patient.

Regeneration of the bone takes time. In order to provide an adequate barrier function, the barrier membrane has to withstand resorption for a sufficient amount of time to allow optimal regeneration of the bone, normally between about 8-12 weeks. In cases of larger, more complex bone defects more time may be required. In such a case, a bioresorbable membrane providing a barrier function for up to 4-6 months is preferably used.

The reinforcement member(s) are preferably adapted to not be resorbed until after 4-6 weeks. Within 4-6 weeks, the regenerated bone is sufficiently stable. Hence, after 4-6 weeks, there is no longer a need for for reinforcement member(s) to stabilize the membrane. However, as the bone has not yet completely regenerated, there is still a demand for a barrier function to avoid tissue ingrowth into the surgical site.
Therefore, in case both parts of the system are bioresorbable, the parts might not be resorbed in the same time, especially, the membrane is preferably not resorbed during these 4-6 weeks.

It is hence desirable that the reinforcement member withstands resorption until a sufficiently stable bone augment situation is built up. Alternatively, the reinforcement member(s) is resorbed at about the same time as the bioresorbable membrane, in case such a membrane is used. A longer stability might e.g. be desired in patients with a slower healing or with larger defects.

Preferably, the membrane is fluid permeable and cell impermeable. Such a membrane allows molecules (nutrients) present e.g. in the blood, which are needed for a neat bone regeneration, to pass the membrane to the treatment site. At the same time, the non-desired invading tissue cells are prevented from entering the treatment site. This ensures proper bone regeneration.

The barrier system according to the invention preferably comprises multiple reinforcement members. With multiple resorbable reinforcement members, the barrier system easily can be applied in the desired shape. Multiple members might be used in a specific covering of the treatment site, especially if larger volumes have to be covered. However, also in case of larger volumes a barrier system with a single reinforcement member with a suitable size and shape can be used. The multiple reinforcement members might be provided in different arrangements depending on the surgical site to be provided to. The reinforcement members might e.g. be provided in an intercrossing manner or in the form of a Y or a combination thereof.

Depending on the volume and shape of the treatment site, different sizes of membranes are used. The membranes preferably have a width to length dimension of between 10 mm/15 mm to 40 mm/45 mm. Hence, typical width/length ratios of membranes are between 2/3 to 8/9. Examples of further typical width/length dimensions are 15 mm/20 mm, 20 mm/30 mm, 25 mm/30 mm, 25 mm/35 mm, 30 mm/35 mm and 35 mm/45 mm. Different ratios and sizes are also possible. The sizes are not to be understood in restricting the shape of the membrane. The membranes are not mandatorily in a rectangular shape but might have any suitable shape.

Preferably, the at least one reinforcement member is plastically deformable. Further, the reinforcement member(s) is preferably not too elastic. Therewith, a preformed structure of the reinforcement member and of the implanted barrier system is kept also under slight loads as can occur during the healing phase, e.g. by soft tissue pressure or tongue movement. The surgeon might bend the member(s) manually into the desired shape and implant the reinforcement member and the reinforced membrane at the treatment site. Alternatively, the user can shape the reinforcement member by adapting it to a model representing the desired anatomical situation of the defect site after bone regeneration. Such a model can be created prior to surgery either by traditional model/cast or CAD/CAM techniques. A patient and site specific CAD/CAM preparation of the already shaped reinforcement member itself is also an option.

The at least one reinforcement member preferably comprises at least one of the following properties:
- Elastic limit of about 160-260 MPa, preferably 190-240 MPa and/or
- Tensile strength of about 220-320 MPa, preferably 240-300 MPa and/or
- Elongation at fracture of about 5-40%, preferably 15-25%.

These parameters have been shown to be especially suitable for resorbable reinforcement members. The proposed parameters allow a reliable and easy bending of the reinforcement member(s) without leading to rupture of the reinforcement member(s). Therewith, the barrier system can reliably be brought into the desired shape. The reinforcement member(s) of the barrier system are at the same time sufficiently stiff against deformation during use.

Preferably, the at least one reinforcement member comprises magnesium, preferably in the range of 96-99.7%, more preferably 98.6-99.5%, zinc, preferably in the range of traces up to 3%, more preferably 0.3-1% and calcium, preferably in the range of traces up to 1%, more preferably 0.2-0.6%. (All values are in wt.%)

Elements which are toxic in small amounts and in particular rare earth metal elements are preferably avoided in compositions for reinforcement members.

Such a magnesium alloy has been shown to be especially suitable to be used as a resorbable reinforcement member. The alloy provides suitable mechanical properties, is biocompatible and is sufficiently slowly resorbed for a stable bone augment situation to be built up.

Preferably, the at least one reinforcement member is free of bioincompatible elements. By avoiding any bioincompatible elements, a risk of intoxication is avoided. In case a metal alloy is used, all components and degradation products of the metal alloy are preferably biocompatible.

The invention further concerns a method of forming a reinforced barrier system, preferably a barrier system as described hereinbefore. The method comprises the steps of:
a) Providing a membrane, preferably a bioresorbable membrane, more preferably a bioresorbable collagen membrane and
b) Providing at least one, preferably multiple, bioresorbable reinforcement members comprising or being made of a bioresorbable polymer, preferably a synthetic polymer and/or a bioresorbable metal, preferably a magnesium alloy.
c) connecting the membrane and the at least one reinforcement member.

Preferably, the at least one reinforcement member is connected by embedding the reinforcement member during formation of the membrane. The reinforcement member might for example be embedded in a reconstituted collagen membrane during a reconstituting step. In an alternative example, a synthetic membrane such as an electro-spun PLGA membrane is used and the reinforcement member embedded during the production.

The at least one bioresorbable reinforcement member is preferably cut from an extruded piece.

Extruding allows providing reinforcement structures which might be cut into the desired thickness. By extruding, sufficient quantities of reinforcement members can be provided in homogeneous quality in little time.

Heat treatment of the material of the reinforcement member or the reinforcement member itself may be used to adjust the degradation rate and mechanical properties of the reinforcement member.

The invention is also directed to a method of regenerating a bone after surgery, comprising the step of using a barrier system, preferably a barrier system as described hereinbefore. The method comprises the steps of:
a) Providing at least one bioresorbable reinforcement member, preferably a reinforcement member as described hereinabove,
b) Adjusting a form of the at least one reinforcement member to a treatment site to be covered by deforming at least one reinforcement member,
c) Implanting the reinforcement member at a treatment site
d) Implanting a membrane, preferably a membrane as described hereinabove on the reinforcement member such that tissue cells are prevented from passing the membrane.
In step a), the at least one bioresorbable reinforcement member might be provided attached to the membrane, e.g. through embedding or mechanical connection. In such a case, step d) is performed simultaneously with step c), respectively.

Optionally a mock up model of the desired anatomical situation after bone regeneration can be used to shape the reinforcement member(s) before implantation, or the reinforcement member can be preshaped by making use of CAD/CAM technologies.

Preferably, bone graft material is provided into the treatment site before covering the treatment site.

The invention is further directed to a bioresorbable reinforcement member for use in a barrier system for guided bone and tissue regeneration. The bioresorbable reinforcement member is preferably a reinforcement member as defined hereinabove in context with the barrier system.

Further aspects of the invention are disclosed with reference to the following schematic figures. The figures schematically show
- Fig. 1:: A barrier system according to the invention;
- Fig. 2:: An alternative barrier system according to the invention;
- Fig. 3:: An alternative barrier system according to the invention;
- Fig. 4:: An alternative barrier system according to the invention;
- Fig. 5:: An alternative barrier system according to the invention;
- Fig. 6:: An alternative barrier system according to the invention;
- Fig. 7:: An alternative barrier system according to the invention;
- Fig. 8:: A cross section trough a barrier system according to the invention;
- Fig. 9:: A cross section trough an alternative barrier system according to the invention;
- Fig. 10:: A cross section trough an alternative barrier system according to the invention;
- Fig.11a,b:: A reinforcement member according to the present invention separated and embedded;
- Fig. 12:: An implanted reinforced barrier system according to the present invention;
- Fig. 13:: A diagram of a method of forming a barrier system according to the present invention.

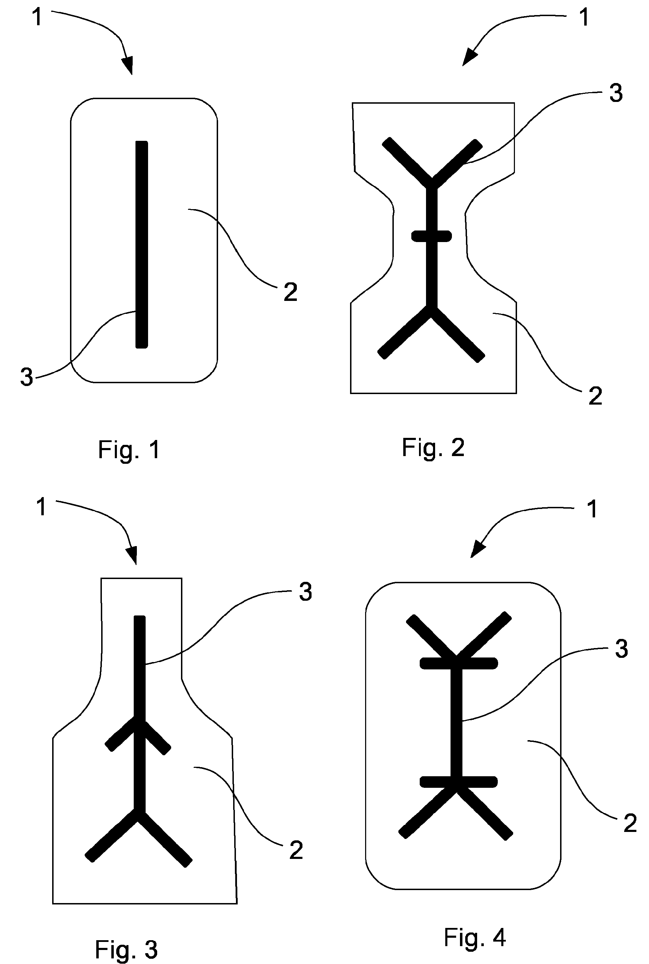

Figure 1 shows a barrier system 1 according to the invention. The barrier system 1 comprises a bioresorbable membrane 2 and a single bioresorbable reinforcement member 3. The bioresorbable membrane 2 is formed of a reconstituted collagen membrane. The collagen fibers of the membrane are crosslinked. The fibers might be crosslinked with sugars or directly through a physical corsslinking. The bioresorbable reinforcement member 3 is made of a magnesium alloy comprising 98.7 wt.% magnesium, 1 wt.% zinc and 0.3 wt.% calcium.

The reinforcement member 3 is embedded into the bioresorbable membrane 2. The reinforcement member (3) is therefore provided between or on a layer of reconstituted collagen fibers and covered with fibers, which are then reconstituted with the layer already reconstituted and themselves. The barrier system 1 has a thickness of 0.3 mm, a length of 30 mm and a width of 20 mm.

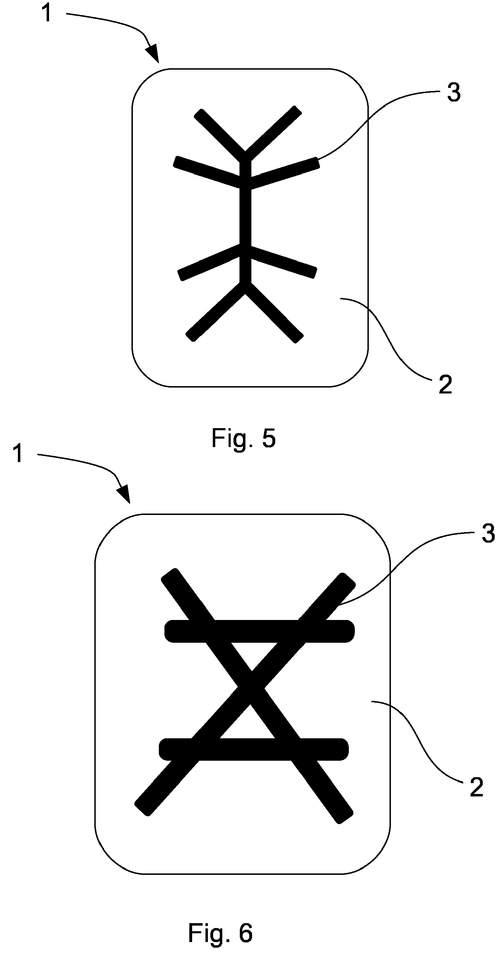

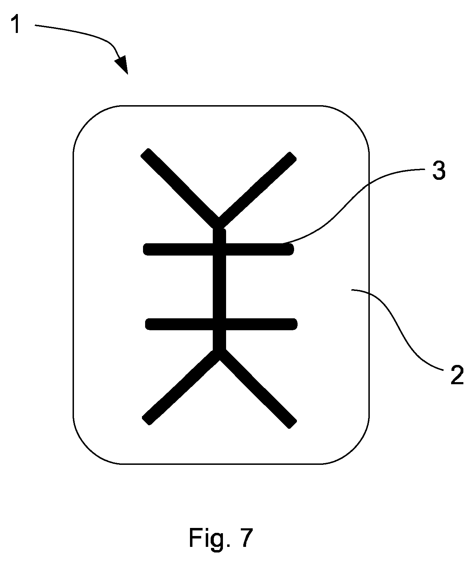

Figures 2-7 disclose different alternatives of barrier systems 1 according to the invention. These embodiments comprise multiple reinforcement members 3 (one reinforcement member in each figure is exemplarily provided with a reference sign). The barrier systems 1 vary in size as well as in number and arrangement of the reinforcement members 3. The dimension of the barrier systems 1 of Figures 2 and 3 are the same as for Figure 1. The smaller side of Figure 2 has a width of 10 mm. The barrier system 1 of Figure 4 has a width of 25 mm and a length of 30 mm; the system 1 of Figure 5 and 6 of 35 mm and 45 mm; the system of Figure 7 of 40 mm and 45 mm. Depending on the dimension of the treatment site, a suitable barrier system 1 is used.

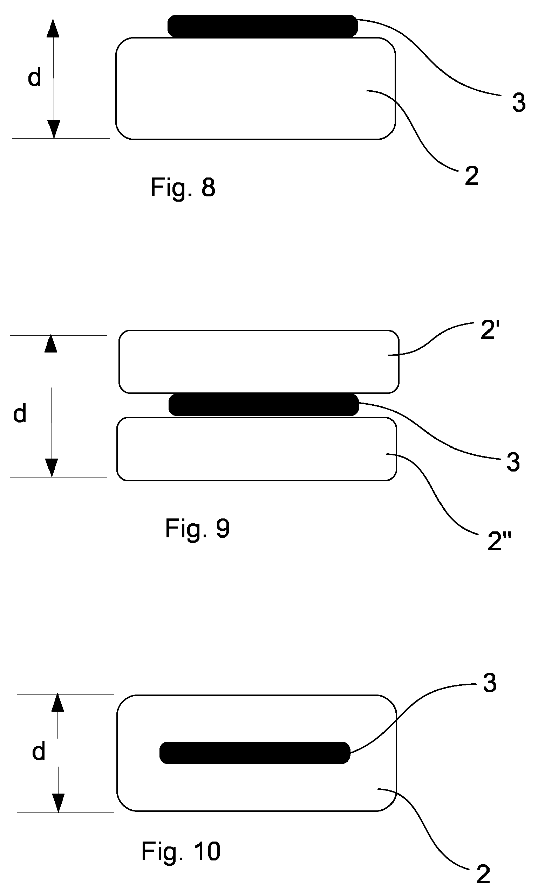

Figure 8 discloses a cross-section through a barrier system 1 according to the invention. The reinforced member 3 is sutured onto a bioresorbable membrane 2. The bioresorbable membrane 2 is a native collagen membrane. The barrier membrane has a thickness d of 0.35 mm.

Figure 9 is a cross-section of an alternative barrier system 1. The reinforcement member 3 is sandwiched between two layers of bioresorbable membranes 2', 2". The two bioresorbable membranes are formed of reconstituted collagen fibers 2' and a synthetic polymer 2". The two layers 2', 2" are not connected to each other and the reinforcement member 3 is glued to both layers. The thickness of the barrier membrane 1 is 0.35 mm.

Figure 10 discloses a cross-section of a further alternative of a reinforced barrier membrane 1 according to the invention. The reinforcement member 3 is embedded in a reconstituted barrier membrane 2. The thickness of the barrier membrane 1 is 0.35 mm.

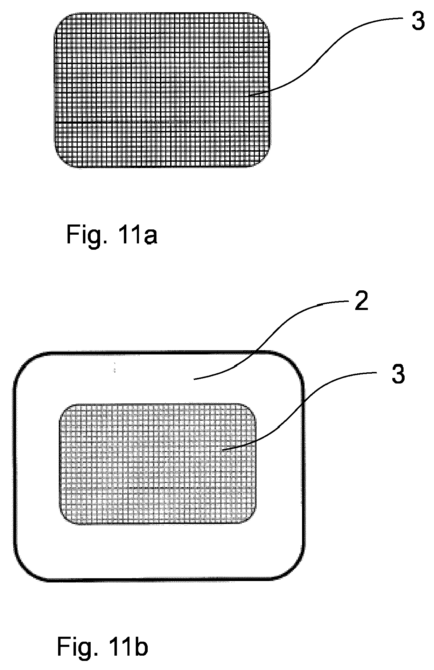

Fig. 11a shows a reinforcement member 3 in the form of a mesh. The mesh has a dimension of width/length of 20 mm/40 mm. The thickness of the mesh is 0.1 mm. the pore-size of the mesh is 0.5 mm.

Fig. 11b shows the reinforcement member 3 of Fig. 11a attached to a membrane 2.

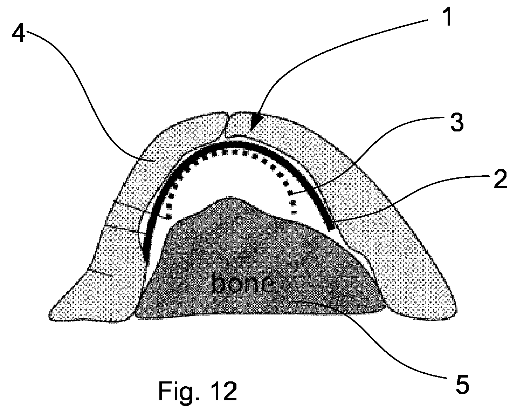

Fig. 12 discloses a barrier system 1 created in situ. The reinforcement member 3 is shaped into the desired form by a dentist and placed on a bone 5. The reinforcement member 3 is covered by the resorbable membrane 2 in situ, i.e. at the treatment site. The membrane is kept in place by fixing it by pins (not shown) to the suitably designed reinforcement member 3. Soft tissue 4 of the patient is placed over the membrane 2. The membrane 2 will be resorbed after four months. The reinforcement member will be resorbed after 4-6 weeks. After 4-6 weeks, the regenerated bone is sufficiently stable to not collapse under the occurring loads. However, the membrane 2 is still needed to keep the barrier function and avoid tissue ingrowth for optimal regeneration of bone in the defect site. There is no need to remove any part of the bioresorbable membrane 2 nor the reinforcement member 3.

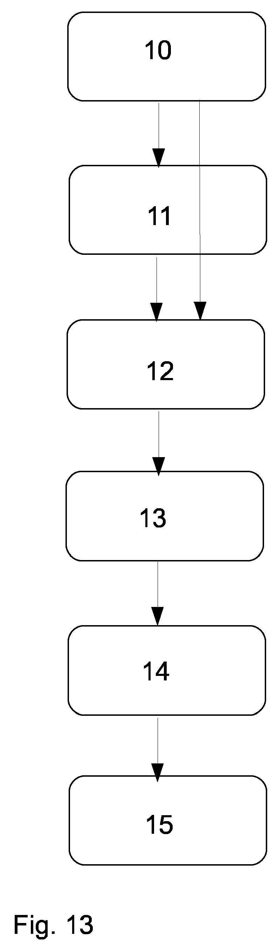

Figure 13 shows a diagram of the steps of forming an embedded reinforcement member according to the invention with the following steps:
10: Deposition of first layer of collagen fibrils
11:(Optional: compaction to desired thickness and density)
12: Placement of reinforcement element on top of first collagen layer
13: Deposition of second layer of collagen fibrils
14: Compression of collagen layer including reinforcement element to desired density and thickness
15: Crosslinking of collagen fibrils to desired degree

The following table discloses compositions for different reinforcement members 3 according to the present invention and their respective parameters.

**[Table 1]**

| 1 < Zn/Ca < 4 | Mg: 98.7 % | Zn: 1% | Ca: 0.3% | Tensile yield strength: 240 MPa | Ultimate tensile strength: 260 MPa | Elongation at fracture: 25% |
|---|---|---|---|---|---|---|
| Zn/Ca > 5 | Mg: 98.25% | Zn: 1.5% | Ca: 0.25% | 225 MPa | 260 MPa | 25% |
| Zn/Ca < 1 | Mg: 99.1% | Zn: 0.45% | Ca: 0.45% | 255 MPa | 264 MPa | 13% |
| Poly-ε-caprolactone (PCL) : | Molecular Weight: 50000 to 100000 Da (84500 Da) | Polydispersity of 1.5 to 1.9 (1.78) | Maximum crystalline fraction: 40-70% | Tensile strength (yield): 15-20 (17) MPa | Elastic (tensile) modulus: 400 - 500 MPa (440 MPa) | Elastic (flexural) modulus: 400 - 500 MPa (414 MPa) |

MgZnCa alloys with different Zn/Ca ratios can be produced with nanometer-sized intermetallic particles (IMPs). These very fine precipitates generate excellent mechanical properties (see Table) due to the generation of a very fine microstructure (grain size ≈ 1 µm). Depending on the Zn/Ca ratio the type of IMPs can be modified. While comparable mechanical properties are achieved for different Zn/Ca ratios (see table), the degradation properties can be significantly tailored because of the different electrochemical properties of the IMPs. MgZnCa alloys with Zn/Ca ratios ≤ 1 (e.g. MgZn0.2Ca0.5) show primarily the ignoble Carich (Mg,Zn)₂Ca phase; in alloys with 1 < Zn/Ca < 4 (e.g. MgZn1Ca0.3) the IMPs (Mg,Zn)₂Ca and Mg₆Zn₃Ca₂ can co-exist; and in alloys with Zn/Ca > 5 (e.g. MgZn1.5Ca0.25) only the Zn-rich Mg₆Zn₃Ca₂ IMPs predominate.

The Poly-ε-caprolactone (PCL) has further properties of an elongation at break of 300-500 % (400 %) and an Elastic (compressive) modulus: 400 - 500 MPa (455 MPa).

## Claims

1. A barrier system (1) for guided bone and tissue regeneration comprising a membrane, preferably a bioresorbable membrane, (2) and at least one bioresorbable reinforcement member (3), **characterized in that** the reinforcement member (3) comprises or is made of a bioresorbable polymer, preferably a synthetic polymer and/or a bioresorbable metal, preferably a magnesium alloy.

2. The barrier system (1) according to claim 1, wherein the membrane (2) is a bioresorbable collagen membrane, preferably a reconstituted collagen membrane.

3. The barrier system (1) according to claim 2, wherein the bioresorbable collagen membrane is collagenase-resistant.

4. The barrier system (1) according to any preceding claim, wherein the at least one reinforcement member (3) is embedded in the membrane.

5. The barrier system (1) according to any preceding claim, wherein the at least one reinforcement member (3) is mechanically connected to the membrane.

6. The barrier system (1) according to any preceding claim, wherein at least the bioresorbable reinforcement member (2) is adapted to not be resorbed before 4-6 weeks in a body of a patient.

7. The barrier system (1) according to any preceding claim comprising multiple reinforcement members (3).

8. The barrier system (1) according to any preceding claim, wherein the at least one reinforcement member (3) is plastically deformable.

9. The barrier system (1) according to any preceding claim, wherein the at least one reinforcement member (3) comprises at least one of the following properties:
- Elastic limit of about 160-260 MPa, preferably 190-240 MPa and/or
- Tensile strength of about 220-320 MPa, preferably 240-300 MPa and/or
- Elongation at fracture of about 5-40%, preferably 15-25%.

10. The barrier system (1) according to any preceding claim, wherein the at least one reinforcement member (3) comprises magnesium, preferably in the range of 96-99.7%, more preferably 98.6-99.5%, zinc, preferably in the range of traces up to 3%, more preferably 0.3-1% and calcium, preferably in the range of traces up to 1%, more preferably 0.2-0.6%. (All values are in wt.%)

11. Method of forming a barrier system (1), preferably a barrier system (1) according to any of the previous claims, comprising the steps of:
a) Providing a membrane, preferably a bioresorbable membrane (2), more preferably a collagen membrane and
b) Providing at least one, preferably multiple, bioresorbable reinforcement member (3) comprising or being made of a bioresorbable polymer, preferably a synthetic polymer and/or a bioresorbable metal, preferably a magnesium alloy.
c) Connecting the at least one membrane and the at least one reinforcement member (3).

12. The method according to claim 1, wherein the at least one reinforcement member (3) is connected by embedding the reinforcement member (3) in the membrane during formation of the membrane.

13. Method of regenerating a bone after surgery, comprising the step of using a barrier system (1), preferably a barrier system (1) according to any of the claims 1 to 10, comprising the steps of:
a) Providing at least one bioresorbable reinforcement member, preferably a reinforcement member as described hereinabove,
b) Adjusting a form of the at least one reinforcement member to a treatment site to be covered by deforming at least one reinforcement member,
c) Implanting the reinforcement member at a treatment site
d) Implanting a membrane, preferably a membrane as described hereinabove on the reinforcement member such that tissue cells are prevented from passing the membrane.

14. Method according to claim 13, wherein in step a), the at least one bioresorbable reinforcement member is provided attached to the membrane, in particular through embedding or mechanical connection, therewith allowing steps c) and d) to be carried out simultaneously.

15. A bioresorbable reinforcement member (3), preferably a bioresorbable reinforcement member (3) as defined in one of the claims 1-10, for use in a barrier system (1) for guided bone and tissue regeneration.
